# EUROPEAN PATENT SPECIFICATION

(11) **EP 4 252 254 B1**
(45) Date of publication and mention of the grant of the patent: **07.01.2026**
(21) Application number: 21834884.5
(22) Date of filing: 29.11.2021
(51) Int. Cl.: G16H 50/20, G16H 40/67

(54) **COMPUTER-IMPLEMENTED METHOD FOR DETECTING A MICROSLEEP STATE OF MIND OF A PERSON BY PROCESSING AT LEAST ONE EEG TRACE USING AN ARTIFICIAL INTELLIGENCE ALGORITHM AND SYSTEM CONFIGURED TO IMPLEMENT SUCH METHOD**
COMPUTER-IMPLEMENTIERTES VERFAHREN ZUR ERKENNUNG DES ZUSTANDES VON MIKROSCHLAF EINER PERSON DURCH VERARBEITUNG MINDESTENS EINER EEG-SPUR MIT EINEM ALGORITHMUS VON KÜNSTLICHER INTELLIGENZ UND SYSTEM, DAS FÜR DIE IMPLEMENTIERUNG EINES SOLCHEN VERFAHRENS KONFIGURIERT IST
PROCÉDÉ MIS EN OEUVRE PAR ORDINATEUR POUR DÉTECTER UN ÉTAT DE MICRO-SOMMEIL D'UNE PERSONNE PAR TRAITEMENT D'AU MOINS UNE TRACE D'EEG À L'AIDE D'UN ALGORITHME D'INTELLIGENCE ARTIFICIELLE ET SYSTÈME CONÇU POUR METTRE EN OEUVRE UN TEL PROCÉDÉ

(30) Priority: 30.11.2020 IT 202000028916
(43) Date of publication of application: 04.10.2023
(73) Proprietor: ORAIGO S.R.L., 35134 Padova (IT)
(72) Inventor: GALETTA, Michele, 35010 San Giorgio Delle Pertiche (PD) (IT); MAGUOLO, Gianluca, 35123 Padova (IT); NANNI, Loris, 48121 Ravenna (IT); ROSSATO, Gianluca, 37036 San Martino Buon Albergo (VR) (IT); TONON, Davide, 37010 Costermano Sul Garda (VR) (IT)
(74) Representative: Marchioro, Paolo
(86) International application number: PCT/IB2021/061073
(87) International publication number: WO 2022/113037

(56) References cited:
- WO-A1-2016/110804
- US-A- 6 070 098
- US-A1- 2020 151 474
- LAPINLAMPI A-M ET AL: "Sleep staging with frontopolar EEG derivation", SLEEP & HYPNOSIS,, vol. 6, no. 2, 31 January 2004 (2004-01-31), pages 59 - 65, XP009102651, ISSN: 1302-1192
- ANONYMOUS: "Activation function - Wikipedia", 23 November 2020 (2020-11-23), XP055829974, Retrieved from the Internet <URL:https://en.wikipedia.org/w/index.php?title=Activation_function&oldid=990306314> [retrieved on 20210803]
- ANONYMOUS: "Convolutional neural network - Wikipedia", 29 July 2019 (2019-07-29), XP055735969, Retrieved from the Internet <URL:https://en.wikipedia.org/w/index.php?title=Convolutional_neural_network&oldid=908472838#Image_recognition> [retrieved on 20201001]

## Description

The present invention relates to a computer-implemented method for detecting a specific state of mind of a person, in particular a microsleep state of mind, by processing at least one EEG trace of the same person by means of an artificial intelligence algorithm.

Furthermore, such computer-implemented method provides for warning said person when said state of mind is detected.

The invention also relates to a system for detecting the aforesaid state of mind of a person by processing the aforesaid EEG trace by means of the aforesaid artificial intelligence algorithm.

As is well known, EEG measures the extracellular current flow generated by the spatio-temporal summation of post-synaptic potentials. These post-synaptic potentials have a duration of between ten and a hundred ms, and a maximum amplitude of 20 mV.

These signals are acquired by placing one or more electrodes on a person's head according to the standard positioning called the "international 10-20 system", which involves, among other things, placing two electrodes, called FP1 and FP2 respectively, in the left frontopolar position and in the right frontopolar position on the scalp of such a person.

It is further known that the EEG signals acquired are affected by multiple artefacts, which may be physiological and extra-physiological in nature. In particular, artefacts of a physiological nature include artefacts due to eye movements, which include, among others, the movement of closing and opening the eyelids.

In detail, these artefacts due to eye movement, in particular, present non-negligible amplitudes and are clearly distinguishable from the rest of the signal, especially in the EEG signals acquired at the aforementioned two positions FP1 and FP2.

For this reason, these signals are usually appropriately filtered in order to eliminate such artefacts therefrom and to process a signal exclusively related to post-synaptic potentials.

From the point of view of the states of mind that can occur in a person, there is the state of mind of drowsiness. In particular, this term generally and in the present context means difficulty in staying awake and, normally, such drowsiness is a transitional phase of the sleep-wake cycle. Drowsiness is characterised by a state of numbness and a reduction in the level of consciousness, which are typical signs that one is about to succumb to sleep.

Drowsiness is manifested by irritability, attention deficit, feeling of heaviness in the eyelids, yawning and rubbing of the eyes.

This condition, which can occur naturally and without any contraindications in most cases, at various stages of a person's day can, however, cause a very dangerous situation when it occurs, particularly when a person is driving a vehicle.

For this reason, a lot of researches have recently been carried out in order to be able to find devices capable of promptly detecting the onset of such a state of mind while a person is driving a motorised vehicle, with the aim of warning that person and bringing him or her out of that state of mind.

However, a first category of devices, in order to discriminate such state of minds, requires the combined use of EEG signals and instruments capable of detecting the body movements of the person driving, such as the inclination of the head. In this case, disadvantageously, these devices are very complex and articulated to be used effectively by one person.

Other categories of devices pertaining to the prior art, although less articulated and simpler to use, are not able to promptly detect such a drowsiness state of mind and risk reporting such a condition too late, thus increasing the likelihood of accidents and dangerous situations.

The document US 6 070 098 A detects micro-sleep events and a partial and complete prolonged eyelid closure. However, this document does not explicitly disclose the detection of events defined by lowering a person's upper eyelid to completely cover the pupil and maintaining said coverage for at least 0.5 seconds. Besides, this document uses (infrared) eye tracking and not EEG data for detecting a partial and complete prolonged eyelid closure. Further, this document is completely silent about EEG data collected at the FP1 and/or FP2 position and does not provide any details with regard to the applied filter procedures.

The document US 2020/151474 A1 teaches the detection of micro-sleep events. However, this document uses a band-pass filter with cut-off frequencies which are far away from the cut-off frequency ranges specified in the present invention.

The present invention intends to overcome the drawbacks of the prior art.

In particular, it is an object of the invention to propose a computer-implemented method for detecting a specific state of mind of a person by processing at least one EEG trace by means of an artificial intelligence algorithm, capable of detecting such state of mind more effectively than systems and devices proposed by the prior art.

A further purpose of the invention is to propose a method capable of detecting a specific state of mind of a person more rapidly than systems and devices proposed by the prior art.

The aforesaid purposes are achieved by the computer-implemented method for detecting a specific state of mind of a person by processing at least one EEG trace by means of an artificial intelligence algorithm, in accordance with the main claim to which reference will be made.

Further characteristics of the method of the invention are described in the dependent claims.

Also part of the invention is a system for detecting said state of mind of a person by processing said EEG trace by means of said artificial intelligence algorithm in accordance with claim 14.

The aforesaid purposes, together with the advantages which will be mentioned below, will be better highlighted during the description of some preferred embodiments of the invention which are given, by way of non-limiting example, with reference to the attached drawings, where:
- fig. 1 shows a schematic representation of a first embodiment of the system of the invention;
- fig. 2 shows a schematic representation of a second embodiment of the system of the invention.

The computer-implemented method of the invention, hereinafter described in a preferred but not limiting embodiment, is intended to detect a specific state of mind of a person by processing at least one EEG trace acquired on the same person, by means of an artificial intelligence algorithm, preferably by means of at least one suitably trained convolutional neural network, even more preferably by means of at least one suitably trained network of the "Temporal Convolutional Network" type.

In the implementation example of the method of the invention discussed below, the specific state of mind of a person intended to be detected corresponds to a state of mind defined as "microsleep", wherein "microsleep" means the physical condition of a person which begins when the upper eyelid of the same person droops until it completely covers the pupil and this coverage is maintained for at least 0.5 seconds.

The aforementioned microsleep condition, identified through experimentation by the inventors, manifests itself as a prelude to the drowsiness state of mind in a person, and the inventive idea of the inventors themselves, underpinning the claimed method, is that of being able to exploit the identification of such a microsleep state of mind in order to promptly implement, as a consequence, appropriate actions to interact with the same person or to initiate further analysis on the latter.

According to the preferred embodiment of the invention, preferably but not necessarily, such detection of the microsleep state of mind is implemented on a person while he/she is driving a vehicle, in order to detect the possible transition from the waking state to the drowsy state of said person, and, consequently, in order to promptly warn the same person driving the vehicle of the imminent intensification of the dangerous situation in which he/she is.

It is well known, indeed, that excessive drowsiness is associated with approximately one fifth of road accidents and is one of the main causes of fatal motorway accidents.

It is not excluded, however, that such detection of the condition of microsleep state of mind could be used for other purposes, such as carrying out actigraphy investigations.

It is well known, in fact, that actigraphy is the instrumental investigation used to assess night sleep. More precisely, actigraphy is a methodology that allows prolonged monitoring of the condition of movement (wakefulness) and marked reduction of movement (sleep) and is a sufficiently objective index of the sleep-wake rhythm. In this context, the detection of the microsleep state of mind by the method of the invention allows the transition from the aforementioned waking condition to the sleeping condition of an individual to be promptly and precisely detected, from which moment the further analyses envisaged by the aforementioned actigraphy methodology can be started.

In order to detect such a microsleep state of mind, the computer-implemented method of the invention comprises first of all a training step of said artificial intelligence algorithm, in particular of said convolutional neural network.

This training step involves, in particular, receiving a set of training EEG traces comprising a plurality of EEG traces acquired on several people by means of special devices equipped with one or more electrodes for acquiring such EEG traces.

More precisely, as far as the acquisition of these traces is concerned, the inventors have established a precise protocol involving several people driving for several driving sessions, specifically seven sessions for each driver, each lasting about 40 minutes.

During each session, the person driving must maintain a constant speed along a pre-determined trace, and at least one EEG trace is acquired on that person at one of the positions on that person's scalp defined by the 10-20 standard, preferably at one of the two frontopolar FP1 or frontopolar FP2 positions on such scalp. Even more preferably, both EEG traces are acquired at the two frontopolar positions FP1 and FP2.

In fact, such positions, advantageously, allow more precise and clearer detection of artefacts due to eye movement.

Even more specifically, the acquisition of the aforesaid EEG traces involves the use of six electrodes, 4 of which are frontal (FP1, FP2, AF7, AF8) and two are positioned on T5 and T6, with the ground positioned at AF8 and the reference positioned at AF7. As the reference is placed at AF7, the signals collected will be: FP1-AF7, FP2-AF7, T5-AF7 and T6-AF7, and to define the signals suitable for input to the convolutional neural network, i.e. the pair FP1-T6 and FP2-T5, the signals will be re-referenced by operating the subtractions: (FP2-AF7) - (T5-AF7) = FP2-T5 and (FP1-AF7) - (T6-AF7) = FP1-T6. The use of reference and ground electrodes placed on the forehead and therefore in a position where no hair is present advantageously allows the electrodes themselves to immediately reach optimum impedance levels.

It is not excluded, however, that according to variant embodiments of the invention the EEG trace used may be acquired at different positions according to the 10-20 standard, for example at positions F3-F7-Fz-F4-F8.

At the same time, during these driving sessions, images of the eyes of that person, in particular of the pupils of the latter, are captured using special glasses or eyetrackers equipped with two precision micro-cameras.

At the end of each session, the acquired EEG traces are compared over time with the acquired images of the person's eyes and the periods of time during that session when the same person's upper eyelid droops until it completely covers the pupil of the eye of interest are noted, where this condition occurs for at least 0.5 seconds.

In this way, it was thus possible to discriminate the portions of the EEG traces during which said physical condition occurred.

These acquisitions of the EEG traces and videos relating to the eyes of a person driving were carried out for nine people. It is not excluded, however, that there may be fewer or more than nine people involved in the trial.

In essence, the inventors, through these experiments, understood the possibility of exploiting the particular waveforms that EEG traces assume, due to the artefacts of eye movement, in particular the movement of closing and opening the eyelids, in order to detect the aforementioned microsleep state of mind, the prelude to the state of drowsiness.

Returning, therefore, to the method of the invention, according to a first preferred embodiment, it envisages receiving such training EEG traces and further envisages receiving the aforementioned annotations, indicating the detection for at least one stretch of them of the aforementioned state of mind, in this case of the microsleep state of mind.

In particular, since the signals relating to the EEG traces are analogue signals, before the latter are input to the artificial intelligence algorithm, a pre-processing step is provided, which according to the preferred embodiment of the invention envisages, first of all, converting the same analogue signals into numerical (or digital) signals. In doing so, a filtering operation of the analogue signal is also provided, before its conversion, in order to eliminate frequency components that could negatively alter the result of subsequent processing.

In particular, according to this preferred embodiment of the invention, the analogue signals relating to the EEG traces are filtered by means of a high-pass filter with a cut-off frequency chosen between 0.001 and 0.05 Hz and/or a low-pass filter with a cut-off frequency chosen between 10 and 25 Hz.

Clearly, when both of these filters are used, a bandpass filter is obtained with a bandwidth between said two cut-off frequency values.

Advantageously, the use of the high-pass filter with the above-mentioned cut-off frequency makes it possible to filter out the background noise of the acquired signal. The low-pass filter, at the above-mentioned cut-off frequency, makes it possible to exclude from the signal all those frequencies due to muscular artefacts, keeping instead the brain frequencies and the frequencies due to ocular artefacts (eyelid movement).

As far as the above-mentioned annotations are concerned, it is envisaged to receive one annotation for each of the above EEG traces as input.

Even more specifically, preferably, each of such annotations, according to the first embodiment of the method described herein, comprises a first vector in turn comprising a number of memory cells equal to the duration of the received EEG trace divided by a predetermined sampling range, wherein in each of said memory cells the presence or absence of the state of mind of a person to be identified is annotated, for each of the sampling ranges of the specific EEG trace segment. The implementation example described herein, preferably but not necessarily, envisages that this sampling range is selected between 5 ms and 50 ms, even more preferably 10 ms.

Even more preferably, each of these memory cells of the above-mentioned first vector is defined by one bit, which may assume the value 1 in case in which the specific state of mind (microsleep) has been detected in the relative 10 ms of the EEG trace, or may assume the value 0 if this state of mind has not been detected.

It is not excluded, however, that according to different embodiments of the invention each of such annotations comprises a single memory cell indicating whether or not in said EEG trace segment the microsleep state of mind to be detected has been detected, at least for a stretch.

The method of the invention, in particular the training step of the method of the invention, subsequently comprises providing as input data to said artificial intelligence algorithm, in particular to said Temporal Convolutional Network, each of the EEG traces, and annotations thereof.

With regard to the artificial intelligence algorithm, according to the invention it terminates with an activation function, preferably a sigmoid activation function, to obtain a classification output signal in which the probability of detecting the microsleep state of mind to be detected is recorded, in the input EEG trace segment.

Finally, the training step of the method of the invention envisages that the artificial intelligence algorithm processes each of the EEG traces, in order to train the artificial intelligence algorithm itself, by comparing the classification output signal obtained by processing, with the annotation relating to the same EEG trace.

With regard to the classification output signal, according to the preferred embodiment of the invention, it comprises a second vector, in turn comprising a number of memory cells equal to the duration of the EEG trace divided by a predetermined sampling range, wherein each of the memory cells records the probability of detection of a person's state of mind at a specific sampling range of the EEG trace.

Also in this case, preferably but not necessarily, this sampling range should be chosen between 5 ms and 50 ms, even more preferably 10 ms.

Even more preferably, each of these memory cells of the aforesaid second vector is defined by 8 bytes ("double float" data type), the value of which varies according to the probability calculated by the artificial intelligence algorithm of detecting or not the microsleep state of mind in that 10 ms stretch.

The use of 8 bytes for each memory cell of the second vector makes it advantageous to obtain double precision data concerning the probability of detecting or not the state of mind of a person in the specific 10 ms range.

It is not excluded, also in this case, that according to different embodiments of the invention this classification output signal may present a different structure, provided that it is able to record the probability of the detection of the specific microsleep state of mind, in the input EEG trace.

In particular, it is not excluded that this size of each memory cell may be different from 8 bytes.

Returning to the first preferred embodiment of the computer-implemented method of the invention, it further comprises a step of classifying one or more EEG traces, acquired on a person, preferably by means of two electrodes arranged respectively in a frontopolar position FP1 and in a frontopolar position FP2 on the scalp of the same person.

As mentioned above, it is not excluded that such one or more EEG traces may be acquired at only one of the above-mentioned positions FP1 or FP2.

The purpose of this classification step is to allow the identification or not of the specific microsleep state of mind in said person.

The classification step, according to the invention, involves, as in the case of the training step, receiving at least one EEG trace and performing a pre-processing step on such EEG trace.

The pre-processing step of the EEG trace to be processed involves performing exactly the same operations as described above for the pre-processing step of the training EEG traces. For this reason, for the sake of simplicity, this description is also referred to for the classification step of the EEG trace to be processed, thus preventing having to repeat all the above-mentioned operations treated extensively above.

The method of the invention therefore envisages providing such EEG traces as input to the artificial intelligence algorithm, in particular to the network of the Temporal Convolutional Network type, clearly after the training thereof to the artificial intelligence algorithm, by means of the training step described above.

The artificial intelligence algorithm trained at this point, according to the invention, processes the EEG traces in such a way as to provide as output a classification output signal of the type described above, for each of said EEG traces. In particular, as seen above, this signal is provided at the output of the aforementioned activation function.

Further, the method of the invention, in particular such classification step, provides for deciding on the detection or non-detection of the aforementioned microsleep state of mind of the person, based on the classification output signals provided by the artificial intelligence algorithm.

According to the first preferred embodiment of the invention, this decision step comprises testing whether the probability of detection of a person's state of mind, indicated in a specific classification output signal, is greater than a predefined threshold, for a predefined minimum period of time.

For example, this minimum period of time could be chosen as a lower limit of 6 tenths of a second; however, it could be chosen as 12 tenths of a second, 18 tenths of a second, etc.

Alternatively, according to a different embodiment of the invention, this classification step, in particular the decision as to whether or not to detect the microsleep state of mind, could be implemented by means of a recurrent neural network arranged downstream of the artificial intelligence algorithm, in particular of the network of the Temporal Convolutional Network type, so that the same recurrent neural network is able to receive as input the classification output signals generated by the artificial intelligence algorithm itself, in particular by the network of the Temporal Convolutional Network type.

The use of such a recurrent network favours the discrimination of what has been defined here as being the microsleep state of mind from what is known to be a so-called blink, i.e. a rapid sequence of closing and opening of the eyelids.

In other words, it could be envisaged, according to such an alternative embodiment of the invention, that the decision as to whether or not to detect the microsleep state of mind in the EEG trace as input to the artificial intelligence algorithm would also be left to an artificial intelligence algorithm, rather than to a "traditional" algorithm.

Finally, according to the first preferred embodiment of the invention, once the method has detected a microsleep state of mind on at least one acquired EEG trace segment, the same method provides for promptly providing a warning signal to the same person, so that, if driving, he/she is prompted to exit such a state of mind and is made to return to a waking state of mind.

Such warning signals could be sound signals, visual signals or tactile signals, such as vibrations or low-intensity electrical signals.

Clearly, the steps of acquiring a person's EEG traces, classifying said EEG trace, deciding on the presence of the microsleep state of mind and subsequently generating a warning signal if so must be carried out in sequence and in real time so as to be able to promptly warn the person driving of the dangerous condition in which he or she finds himself or herself. A second preferred embodiment of the method of the invention envisages all the steps already extensively described for the above-mentioned first embodiment, except for the differences described below.

In particular, both during the training step and during the classification step, the method of the invention according to said second preferred embodiment provides that said pre-processing step also comprises a subdivision of each of the EEG traces, preferably once converted to digital, into time segments of a predetermined duration **X.** According to this exemplary embodiment described herein, preferably but not necessarily, this predetermined duration **X** is chosen in the range comprised between 1 second and 15 seconds, even more preferably it is chosen as 8 seconds.

Advantageously, the choice of such a time value of 8 seconds for the predetermined duration **X** allows a compromise between the reduced processing time of the EEG trace segment and the guarantee of an adequate detection of the possible state of mind of a person to be detected in the same EEG trace segment.

It is not excluded, however, that a different value may be chosen as such predetermined duration **X.**

In addition, preferably but not necessarily, this division into time segments requires that each pair of adjacent segments of the EEG trace overlap for a time duration equal to half of said predetermined duration **X;** thus, in this case, each pair of adjacent segments has an overlap of 4 seconds. Also in this respect, it is not excluded that according to different embodiments of the invention such overlap is not provided for or that the time of such overlap is different from half of the above-mentioned predetermined duration **X.**

Further, the method of the invention, according to this second preferred embodiment, provides that during this pre-processing step each EEG trace segment is converted from the time domain to the frequency domain and is represented as a two-dimensional digital image.

With regard to the conversion in the frequency domain, it is preferably carried out using a Fourier transform-based algorithm, in particular the Fast Fourier Transform (FFT).

More precisely, this Fourier transform-derived image is preferably obtained by taking into account, during the transformation, the frequencies of 0.1 Hz, 0.25 Hz, 0.5 Hz and all frequencies of integer value from 1 to 25 Hz.

In particular, this selection of frequency values for the execution of the Fourier transform makes it possible to maintain unchanged the components of the signal characteristic of the eye movement, in particular of the eye movement that occurs during the aforementioned microsleep state of mind, and at the same time makes it possible to filter out from the signal all those components not linked to this eye movement.

It is not excluded that, according to alternative embodiments of the method of the invention, the number and value of the frequencies considered for implementing the Fourier transform may vary from those indicated above for the first preferred embodiment of the invention.

Returning to the aforementioned second preferred embodiment of the method of the invention, with respect to representing the aforementioned transformed information as a two-dimensional image, said image preferably is a two-dimensional grey-scale digital image of size 28x154 pixels, wherein each pixel is represented by 4 bytes ("float" data type).

The particular size of the image is chosen according to the following logic: the number of rows, i.e. 28 rows, is chosen equal to the number of the aforementioned frequencies considered during the Fourier transform, while the number of columns, i.e. 154 columns, is the number of windows into which the signal was divided in the time domain relative to the EEG trace segment, on each of which this transformation is performed. As for the choice that each pixel is represented with 4 bytes, this is advantageously due to the precision of the data that each pixel represents.

It follows from this that, according to different embodiments of the method of the invention, depending on the choice of frequencies considered during the Fourier transform and depending on the number of said chosen subdivision windows, the size of the image may also vary accordingly.

It is also not excluded that the size of each pixel may also be chosen to be different from 4 bytes.

Similarly to the first preferred embodiment of the invention, both the first vector identifying each annotation and the second vector defining each classification signal have a number of memory cells equal to said predetermined duration **X** divided by said predetermined sampling range.

However, in the case of the second preferred embodiment, the value of this sampling range is chosen between 50 ms and 150 ms, even more preferably 100 ms.

Therefore, in the case of the second preferred embodiment of the method of the invention, since, as seen above, said predetermined duration of each EEG trace segment is chosen to be 8 seconds, said first vector will comprise 80 memory cells (8 seconds/100 ms), each therefore relating to a stretch of such EEG trace segment having a duration of 100 ms.

The method of the invention, according to the second preferred embodiment, therefore provides that each EEG trace segment is input to the aforementioned artificial intelligence algorithm, in particular to a convolutional neural network, each in the form of a two-dimensional digital image.

With regard to the architecture of the artificial intelligence algorithm, in particular of the aforementioned at least one convolutional neural network, preferably but not necessarily, it sequentially provides:
- a first convolutional block, in turn comprising in sequence a convolutional layer with 32 4x4 filters, a ReLU (Rectified Linear Units) layer and a normalisation layer;
- a second convolutional block, in turn comprising in sequence a convolutional layer with 64 4x4 filters, a ReLU (Rectified Linear Units) layer, a normalisation layer and a 20% dropout layer;
- a pooling layer of size 2x2 and pitch 2;
- a third convolutional block, in turn comprising in sequence a convolutional layer with 64 4x4 filters, a ReLU (Rectified Linear Units) layer and a normalisation layer;
- a fourth convolutional block, in turn comprising in sequence a convolutional layer with 64 4x4 filters, a ReLU (Rectified Linear Units) layer, a normalisation layer and a 20% dropout layer;
- a fifth convolutional block, in turn comprising in sequence a convolutional layer with 32 3x3 filters, a ReLU (Rectified Linear Units) layer and a normalisation layer;
- a first FC (Full Connection) block equipped in sequence with a FC layer of 128 neurons and a ReLU (Rectified Linear Units) layer;
- a second FC (Full Connection) block with 80 neurons, which defines the above-mentioned activation function, in particular the sigmoid activation function.

Clearly, as indicated above, the architecture of the artificial intelligence algorithm just described is a preferred but not limiting embodiment. In other words, different embodiments of the method of the invention could comprise an artificial intelligence algorithm having a different architecture, provided it is capable of detecting the aforementioned microsleep state of mind.

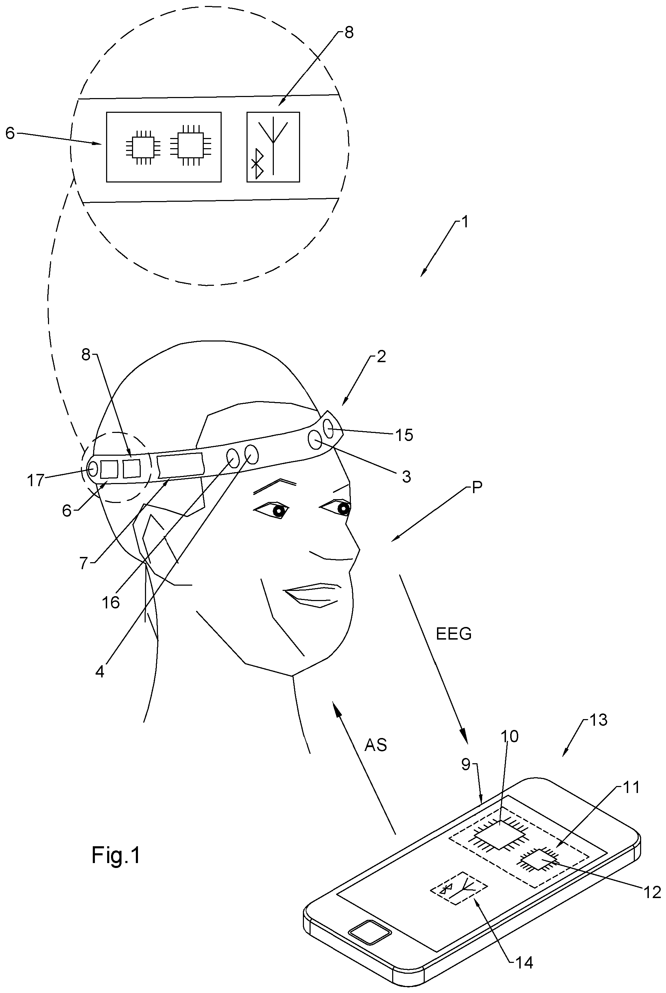

As mentioned above, part of the invention is also the system 1 for detecting the state of mind of a person by processing at least one EEG trace of said person, a first preferred embodiment of which is shown in Fig. 1.

In particular, according to the preferred embodiment of the invention, the system 1 is configured to detect the presence of the microsleep state of mind of a person and in the positive case it is configured to emit a warning signal to said person so as to promptly signal to the latter the dangerous condition in which he or she is, if he or she is driving a vehicle.

According to the invention, the system **1** comprises a device wearable by a person **P,** indicated in Fig. 1 by **2,** so that the same wearable device **2** is placed at least at the frontal part of the scalp of such person **P**. Preferably but not necessarily, such wearable device **2** has an annular shape so that it can be worn on the head of such person.

The preferred embodiment of the wearable device **2** pertaining to the system **1** of the invention comprises two electrodes **3** and **4** for acquiring the EEG trace, configured to be arranged in contact with the scalp of a person **P**, in the frontopolar positions FP1 and FP2. Furthermore, according to the preferred embodiment of the invention, the wearable device **2** belonging to the system **1** comprises six electrodes of which four are front ones (FP1 shown in Fig. 1 with **3**, FP2 shown with **4**, AF7 shown with **15**, AF8 shown with **16**) and two are positioned at T5, not visible in Fig. 1, and T6 shown in Fig. 1 with **17**, with the ground positioned at AF8 and the reference positioned at AF7. As the reference is placed at AF7, the signals collected will be: FP1-AF7, FP2-AF7, T5-AF7 and T6-AF7, and to define the signals suitable for input to the convolutional neural network, i.e. the pair FP1-T6 and FP2-T5, the signals will be re-referenced by operating the subtractions: (FP2-AF7) - (T5-AF7) = FP2-T5 and (FP1-AF7) - (T6-AF7) = FP1-T6. The use of reference and ground electrodes placed on the forehead and therefore in a position where no hair is present, advantageously allows the electrodes themselves to immediately reach optimum impedance levels.

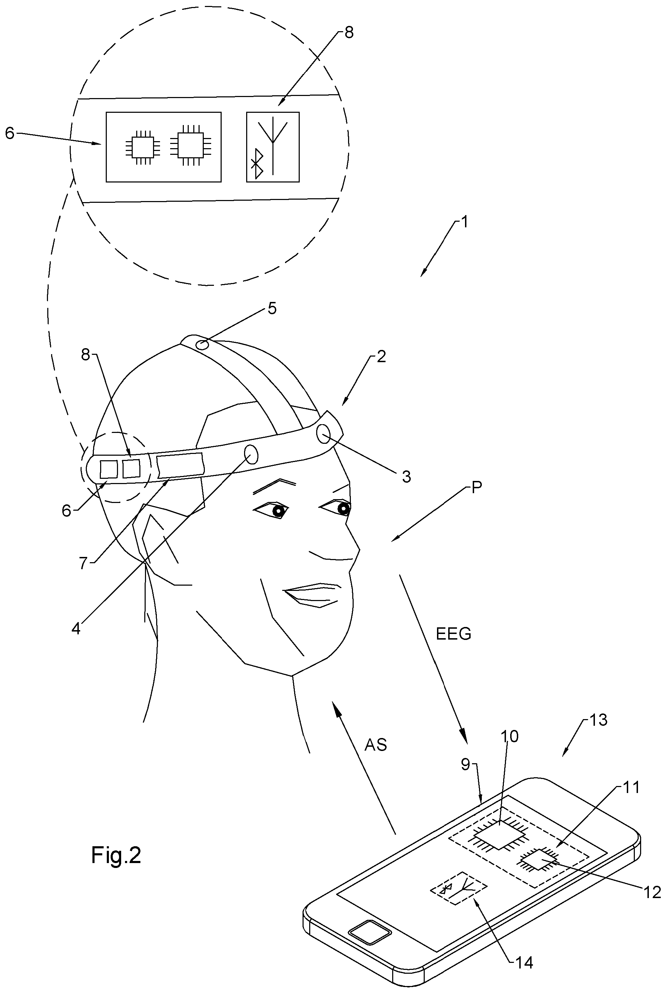

Alternatively, as shown in Fig. 2, the device may comprise the aforementioned two electrodes **3** and **4** for acquiring the EEG trace, configured to be placed in contact with the scalp of a person **P,** in the frontopolar positions FP1 and FP2, and in addition the same device may comprise a third reference electrode **5** suitable for being placed in contact with the scalp of said person in the position CZ.

Also in this case, the wearable device **2** comprises an additional electrode known as the bias or ground electrode.

According to both of these embodiments, preferably, all the electrodes are dry electrodes, also called "of the Dry type", i.e. they do not require the application of a conductive paste between the electrode itself and a person's scalp. It is not excluded, however, that the electrodes themselves may be wet, also called of the "Wet" type, i.e. requiring the application of such a conductive paste.

Further, it is not excluded that according to different embodiments of the invention the wearable device **2** is provided with a single electrode configured to be arranged in contact with a person's scalp in the frontopolar position FP1 or, alternatively, in the frontopolar position FP2, in addition to clearly being provided with said reference electrode.

Further, it is not excluded that such a wearable device may provide, in addition or as an alternative to the two electrodes configured to be placed in contact with a person's scalp in the frontopolar positions FP1 and FP2, additional electrodes configured to acquire EEG traces in different positions of the same person's scalp, such as in positions F3-F7-Fz-F4-F8.

According to the preferred embodiment of the invention, said wearable device **2** also comprises its own control logic unit **6,** configured to receive from the electrodes **3** and **4** said EEG traces and to perform their conversion from analogue EEG traces into digital EEG traces (ADC conversion).

In addition, the wearable device **2** is also equipped with warning means **7**, the usefulness of which will be described in detail shortly.

Further, the preferred embodiment of the invention envisages that the wearable device **2** is provided with first wireless communication means **8**, capable of enabling communication of said wearable device **2** with an external electronic device.

In particular, the control logic unit **6** of the wearable device **2,** once said EEG traces have been acquired and converted into digital format, is configured to transmit said traces to said external device, via such first wireless communication means **8.**

It is not excluded that, according to different embodiments from the preferred one, such first wireless communication means **8** are not present. Indeed, as will be made clear shortly, for such alternative embodiments, the wearable device **2** is to all intents and purposes a stand-alone device.

Returning to the preferred embodiment of the invention, the system **1** further comprises an electronic control unit **9** comprising, in turn, storage means **10** in which the aforementioned artificial intelligence algorithm, presented during the description of the method of the invention, is stored. Furthermore, the electronic control unit **9** comprises processing means **11**, preferably a microprocessor **12**, configured to execute such an artificial intelligence algorithm.

According to the preferred embodiment of the invention, said electronic control unit **9** belongs to a mobile device **13**, such as for example a smartphone or a tablet, equipped in turn with second wireless communication means **14**, configured to establish a wireless communication with the first wireless communication means **7** of the wearable device **2**, in order to receive said EEG traces acquired by means of the same wearable device **2**.

In this case, such first and second wireless communication means **8** and **14** are proximity wireless communication means, e.g. they may implement Bluetooth or BLE communication technology.

An alternative embodiment of the system **1** of the invention could envisage, on the other hand, that said electronic control unit **9** belongs to a remote server, not shown in the figure. In this case, such first and second wireless communication means **8** and **14** enable an internet connection to be established between the server and the wearable device **2**.

Further, according to a third alternative embodiment of the invention, this electronic control unit **9** could correspond to the control logic unit **6** of the wearable device **2**. Therefore, in such a case, as mentioned above, the wearable device **2** is capable of implementing the method of the invention independently, and it could be devoid of the aforementioned first wireless communication means **8**, thus implementing a stand-alone type device.

As described above, when the aforesaid artificial intelligence algorithm is placed in execution and receives input EEG traces from the wearable device **2**, it is configured to perform the aforesaid training step, the classification step or both, which have been described at length above.

This electronic control unit **9,** in the case of performing the classification step by means of the aforementioned suitably trained artificial intelligence algorithm, is also configured to carry out a decision-making step relative to the identification or not of the state of mind, in the specific case of the microsleep state of mind, of the person, based on the classification output signals generated by the artificial intelligence algorithm itself. This decision-making step can be implemented according to the two alternatives described above.

Once the electronic control unit **9** has carried out this decision-making step and has opted for the detection of the aforementioned microsleep state of mind, the same electronic control unit **9** is configured to send a warning signal **AS** to the wearable device **2,** by means of the aforementioned first and second wireless communication means **8** and **14**. Following receipt of the aforementioned warning signal **AS**, the wearable device **2** is configured to signal, via the aforementioned warning means **7**, the dangerous condition which the person wearing the same wearable device **2** is in.

Preferably, such warning means **7** comprise audible warning and/or visual warning and/or tactile warning means, such as vibrations or low-intensity electrical signals.

As mentioned above, this sequence of operations is carried out in real time and is extremely effective if said person is driving a vehicle and enters the aforementioned microsleep state of mind, the prelude to the state of drowsiness.

It is not excluded, however, that according to different embodiments of the system **1** of the invention the wearable device **2** is not provided with the aforementioned warning means **7**, and the classification step and the subsequent step of detecting the microsleep state of mind are used to promptly and precisely detect the transition from the waking condition to the sleeping condition of a person, from which moment it is possible to start, for example, the further analyses envisaged by the aforementioned actigraphy methodology.

To this end, the wearable device **2** must necessarily be equipped with the two electrodes **3** and **4** suitable to be placed in the frontopolar position FP1 and FP2, as well as with two additional electrodes, not shown in the figure, suitable to be placed in the occipital positions O1 and O2, and furthermore with a pulse oximeter.

Furthermore, according to variant embodiments of the invention, the wearable device **2** could also be equipped with an IMU (Inertial measurement unit) in order to improve the performance of detecting the specific state of mind, in particular the microsleep, by detecting movements related to the sagging of a person's head.

Additionally, the wearable device **2** could be equipped with a sensor to measure electrodermal activity, which could detect whether the user is "sweating" and then warn him/her of the need to dry the skin below the electrodes in order to improve the quality of the signals collected.

According to the above, the computer-implemented method and the system adapted to implement said method achieve all the prefixed aims.

In particular, it is achieved the aim to propose a computer-implemented method for detecting a specific state of mind of a person by processing at least one EEG trace by means of an artificial intelligence algorithm, capable of detecting such state of mind more effectively than systems and devices proposed by the prior art.

A further purpose achieved by the invention is to propose a method capable of detecting a specific state of mind of a person more rapidly than systems and devices proposed by the prior art.

## Claims

1. A computer-implemented method for detecting a microsleep state of mind of a person, wherein microsleep means the physical condition of a person that begins when the upper eyelid of a person is lowered to completely cover the pupil and said coverage is maintained for at least 0.5 seconds, and to warn said person in the positive case, said detection being implemented by processing at least one EEG trace of said person by means of at least one suitably trained convolutional neural network, **wherein**:
said method includes a training step comprising the following operations:
- receiving a set of training EEG traces comprising a plurality of EEG traces acquired on more than one person by at least one electrode placed in the frontopolar position FP1 or FP2 on the scalp of said more than one person;
- a pre-processing step of said training EEG traces comprising filtering said training EEG traces by means of a high-pass filter with a cut-off frequency chosen between 0.001 and 0.05 Hz and/or a low-pass filter with a cut-off frequency chosen between 10 and 25 Hz;
- receiving, for each of said training EEG traces, an annotation indicating the identification for at least one portion of said training EEG trace of said microsleep state of mind intended to be detected;
- providing each of said training EEG traces and said relative annotation as input data of said convolutional neural network, said convolutional neural network ending with a sigmoid activation function, to obtain a classification output signal in which the probability of the detection of said microsleep state of mind in said input training EEG trace is recorded;
- by means of said convolutional neural network, processing each of said training EEG traces and training said convolutional neural network, comparing said classification output signal with the annotation relating to said training EEG trace;
**and wherein** said method also comprises a step of classifying at least one EEG trace acquired on a person by at least one electrode arranged in a frontopolar position FP1 or FP2 on the scalp of said person, so as to detect or
not said microsleep state of mind in said person, said classification step comprising the following operations:
- receiving at least said EEG trace;
- a pre-processing step of said EEG trace comprising filtering said EEG trace by means of a high-pass filter with a cut-off frequency chosen between 0.001 and 0.05 Hz and/or a low-pass filter with a cut-off frequency chosen between 10 and 25 Hz;
- providing said EEG trace as input to said convolutional neural network, after said convolutional neural network has been trained;
- processing said EEG trace by means of said already trained convolutional neural network;
- receiving said classification output signal for said EEG trace from said activation function, and deciding whether or not to detect said microsleep state of mind of said person based on said classification output signals.

2. A computer-implemented method according to claim 1, **wherein** said classification step, in particular said decision on whether or not to detect said microsleep state of mind of said person, is based on said classification output signals obtained by processing said EEG trace acquired by two electrodes arranged in a frontopolar position FP1 and in a frontopolar position FP2 on the scalp of said person.

3. A computer-implemented method according to any one of the preceding claims, **wherein** said convolutional neural network is a network of the "Temporal Convolutional Network" type.

4. A computer-implemented method according to any one of the preceding claims, **wherein** said operation of deciding whether or not to detect said microsleep state of mind of a person is implemented by at least one recurrent neural network arranged downstream of said at least one convolutional neural network, so that said recurrent neural network receives as input said classification output signals generated by said convolutional neural network.

5. A computer-implemented method according to any one of the preceding claims, **wherein** said operation of deciding whether or not to detect said microsleep state of mind of a person in said EEG trace, envisages verifying whether the likelihood of detecting said microsleep state of mind indicated in said classification output signal is greater than a pre-set threshold for a predefined minimum period of time.

6. A computer-implemented method according to any one of the preceding claims, **wherein** each of said annotations of said training EEG traces comprises a first vector comprising a number of memory cells equal to the duration of each of said training EEG traces divided by a pre-set sampling range, where in each of said memory cells the presence or absence of said microsleep state of mind of a person is noted for each of the sampling ranges of said training EEG trace.

7. A computer-implemented method according to any one of the preceding claims, **wherein** said classification output signal comprises a second vector comprising a number of memory cells equal to the duration of each of said EEG traces divided by a pre-set sampling range, where in each of said memory cells the likelihood of identifying said microsleep state of mind of a person at a specific sampling range of said EEG trace is recorded.

8. A computer-implemented method according to any one of claims 6 or 7, **wherein** said sampling range is comprised between 5 ms and 50 ms, preferably said sampling range is chosen to be 10 ms.

9. A system (1) for detecting the state of mind of a person (P) by processing at least one EEG trace of said person (P) according to the method according to any one of the preceding claims, **wherein** it comprises:
- a device (2) wearable by said person (P) so that said wearable device (2) is placed at least at the front part of the scalp of said person (P), said wearable device (2) being provided with at least one electrode (3, 4) adapted to be placed in contact with the scalp of said person (P) in the frontopolar position FP1 or FP2 of the scalp itself of said person (P), said wearable device (2) being configured to acquire at least said EEG trace of said person (P) by means of said electrode (3, 4);
- an electronic control unit (9) comprising storage means (10) in which a convolutional neural network is stored according to any one of the preceding claims and processing means (11) configured to execute said convolutional neural network, said convolutional neural network being configured to execute:
- said training step, when said convolutional neural network receives as input said training EEG traces and annotations thereof; and/or
- said classification step, when said convolutional neural network receives as input at least one EEG trace of said person (P) so as to detect or not said specific microsleep state of mind in said person;
- warning means, configured to warn said person of the detection of said microsleep state of mind when said electronic control unit detects said microsleep state of mind of said person.

10. A system (1) according to claim 9, **wherein** said wearable device is provided with first wireless communication means (8), said electronic control unit (9) belonging to a device (13) distinct from said wearable device (2) and provided with second wireless communication means (14), said wearable device (2) being configured, once said at least one EEG trace is acquired, to transfer said at least one EEG trace to said electronic device (13) by said first and second wireless communication means (8, 14).

## Patentansprüche

1. Computerimplementiertes Verfahren zum Erkennen eines Mikroschlaf-Geisteszustands einer Person, wobei Mikroschlaf den körperlichen Zustand einer Person bezeichnet, der beginnt, wenn das obere Augenlid einer Person abgesenkt und die Pupille vollständig bedeckt wird, und die besagte Bedeckung für mindestens 0,5 Sekunden dauert, und im positiven Fall zum Warnen der besagten Person, wobei die besagte Erkennung durch Verarbeiten mindestens einer EEG-Spur der besagten Person mittels mindestens eines angemessen trainierten Convolutional Neural Networks implementiert wird, **wobei**:
das besagte Verfahren einen Trainingsschritt umfasst, der die folgenden Vorgänge umfasst:
- Empfangen eines Satzes von EEG-Trainingsspuren, der eine Vielzahl von EEG-Spuren umfasst, die bei mehr als einer Person durch mindestens eine Elektrode, die in der frontopolaren Position FP1 oder FP2 auf der Kopfhaut der besagten mehr als einen Person platziert wird, erfasst werden;
- einen Vorverarbeitungsschritt der besagten EEG-Trainingsspuren, der das Filtern der besagten EEG-Trainingsspuren mittels eines Hochpassfilters mit einer ausgewählten Grenzfrequenz zwischen 0,001 und 0,05 Hz und/oder eines Tiefpassfilters mit einer ausgewählten Grenzfrequenz zwischen 10 und 25 Hz umfasst;
- Empfangen einer Annotation für jede der besagten EEG-Trainingsspuren, die die Identifizierung für mindestens einen Teil der besagten EEG-Trainingsspur des besagten Mikroschlaf-Geisteszustands angibt, der erkannt werden soll;
- Bereitstellen jeder der besagten EEG-Trainingsspuren und der besagten relativen Annotation als Eingabedaten des besagten Convolutional Neural Networks, wobei das besagte Convolutional Neural Network mit einer Sigmoidaktivierungsfunktion endet, um ein Klassifizierungsausgangssignal zu erhalten, in dem die Wahrscheinlichkeit der Erkennung des besagten Mikroschlaf-Geisteszustands in der besagten eingegebenen EEG-Trainingsspur aufgezeichnet ist;
- mittels des besagten Convolutional Neural Networks, Verarbeiten jeder der besagten EEG-Trainingsspuren und Trainieren des besagten Convolutional Neural Networks, wobei das besagte Klassifizierungsausgangssignal mit der Annotation in Bezug auf die besagte EEG-Trainingsspur vergleicht wird;
**und wobei** das besagte Verfahren auch einen Schritt des Klassifizierens mindestens einer EEG-Spur umfasst, die bei einer Person durch mindestens eine Elektrode erfasst wird, die in einer frontopolaren Position FP1 oder FP2 auf der Kopfhaut der besagten Person angeordnet ist, um den besagten Mikroschlaf-Geisteszustand der besagten Person zu erkennen oder nicht,
wobei der besagte Klassifizierungsschritt die folgenden Vorgänge umfasst:
- Empfangen mindestens der besagten EEG-Spur;
- einen Vorverarbeitungsschritt der besagten EEG-Spur, der das Filtern der besagten EEG-Spur mittels eines Hochpassfilters mit einer ausgewählten Grenzfrequenz zwischen 0,001 und 0,05 Hz und/oder eines Tiefpassfilters mit einer ausgewählten Grenzfrequenz zwischen 10 und 25 Hz umfasst;
- Bereitstellen der besagten EEG-Spur als Eingabe in das besagte Convolutional Neural Network, nachdem das besagte Convolutional Neural Network trainiert wurde;
- Verarbeiten der besagten EEG-Spur mittels des besagten bereits trainierten Convolutional Neural Networks;
- Empfangen des besagten Klassifizierungsausgangssignals für die besagte EEG-Spur von der besagten Aktivierungsfunktion und Entscheiden, ob der besagte Mikroschlaf-Geisteszustand der besagten Person basierend auf den besagten Klassifizierungsausgangssignalen erkannt werden soll oder nicht.

2. Computerimplementiertes Verfahren nach Patentanspruch 1, **wobei** der besagte Klassifizierungsschritt, insbesondere die besagte Entscheidung, ob der besagte Mikroschlaf-Geisteszustand der besagten Person erkannt werden soll oder nicht, auf den besagten Klassifizierungsausgangssignalen basiert, die durch Verarbeiten der besagten EEG-Spur erhalten werden, die von zwei Elektroden erfasst wird, die in einer frontopolaren Position FP1 und in einer frontopolaren Position FP2 auf der Kopfhaut der besagten Person angeordnet sind.

3. Computerimplementiertes Verfahren nach jeglichem der vorhergehenden Patentansprüche, **wobei** das besagte Convolutional Neural Network ein Netzwerk vom Typ "Temporal Convolutional Network" ist.

4. Computerimplementiertes Verfahren nach jeglichem der vorhergehenden Patentansprüche, **wobei** der besagte Vorgang des Entscheidens, ob der besagte Mikroschlaf-Geisteszustand einer Person erkannt werden soll oder nicht, durch mindestens ein rekurrentes neuronales Netz implementiert wird, das nach dem besagten mindestens einen Convolutional Neural Network angeordnet ist, sodass das besagte rekurrente neuronale Netz als Eingabe die besagten Klassifizierungsausgangssignale empfängt, die durch das besagte Convolutional Neural Network erzeugt werden.

5. Computerimplementiertes Verfahren nach jeglichem der vorhergehenden Patentansprüche, **wobei** der besagte Vorgang des Entscheidens, ob der besagte Mikroschlaf-Geisteszustand einer Person in der besagten EEG-Spur erkannt werden soll oder nicht, das Verifizieren vorsieht, ob die Wahrscheinlichkeit des Erkennens des besagten Mikroschlafzustands, der im besagten Klassifizierungsausgangssignal angegeben ist, größer als ein voreingestellter Schwellenwert für einen vordefinierten Mindestzeitraum ist.

6. Computerimplementiertes Verfahren nach jeglichem der vorhergehenden Patentansprüche, **wobei** jede der besagten Annotationen der besagten EEG-Trainingsspuren einen ersten Vektor umfasst, der eine Anzahl von Speicherzellen umfasst, die gleich der Dauer jeder der besagten EEG-Trainingsspuren dividiert durch ein voreingestelltes Abtastintervall ist, wobei in jeder der besagten Speicherzellen das Vorliegen oder Nichtvorliegen des besagten Mikroschlaf-Geisteszustand einer Person für jeden der Abtastintervalle der besagten EEG-Trainingsspur annotiert ist.

7. Computerimplementiertes Verfahren nach jeglichem der vorhergehenden Patentansprüche, **wobei** das besagte Klassifizierungsausgangssignal einen zweiten Vektor umfasst, der eine Anzahl von Speicherzellen umfasst, die gleich der Dauer jeder der besagten EEG-Spuren dividiert durch ein voreingestelltes Abtastintervall ist, wobei in jeder der besagten Speicherzellen die Wahrscheinlichkeit, den besagten Mikroschlaf-Geisteszustand einer Person in einem bestimmten Abtastintervall der besagten EEG-Spur zu identifizieren, aufgezeichnet ist.

8. Computerimplementiertes Verfahren nach jeglichem der Patentansprüche 6 oder 7, **wobei** das besagte Abtastintervall zwischen 5 ms und 50 ms liegt, vorzugsweise wird das besagte Abtastintervall mit 10 ms gewählt.

9. System (1) zum Erkennen des Geisteszustands einer Person (P) durch Verarbeiten mindestens einer EEG-Spur der besagten Person (P) gemäß dem Verfahren nach jeglichem der vorhergehenden Patentansprüche, **wobei** das System Folgendes umfasst:
- eine Vorrichtung (2), die von der besagten Person (P) getragen werden kann, sodass die besagte tragbare Vorrichtung (2) mindestens am vorderen Teil der Kopfhaut der besagten Person (P) platziert wird, wobei die besagte tragbare Vorrichtung (2) mit mindestens einer Elektrode (3, 4) versehen ist, die ausgelegt ist, um in Kontakt mit der Kopfhaut der besagten Person (P) in der frontopolaren Position FP1 oder FP2 der Kopfhaut selbst der besagten Person (P) platziert zu werden, wobei die besagte tragbare Vorrichtung (2) konfiguriert ist, um mindestens die besagte EEG-Spur der besagten Person (P) mittels der besagten Elektrode (3, 4) zu erfassen;
- eine elektronische Steuereinheit (9), umfassend Speichermittel (10), in denen ein Convolutional Neural Network nach jeglichem der vorhergehenden Patentansprüche gespeichert ist, und Verarbeitungsmittel (11), die konfiguriert sind, um das besagte Convolutional Neural Network auszuführen, wobei das besagte Convolutional Neural Network konfiguriert ist, um Folgendes auszuführen:
- den besagten Trainingsschritt, wenn das besagte Convolutional Neural Network als Eingabe die besagten EEG-Trainingsspuren und Annotationen davon empfängt; und/oder
- den besagten Klassifizierungsschritt, wenn das besagte Convolutional Neural Network als Eingabe mindestens eine EEG-Spur der besagten Person (P) empfängt, sodass der besagte spezifische Mikroschlaf-Geisteszustand der besagten Person erkannt wird oder nicht;
- Warnmittel, die dazu konfiguriert sind, die besagte Person bei Erkennen des besagten Mikroschlaf-Geisteszustand zu warnen, wenn die besagte elektronische Steuereinheit den besagten Mikroschlafzustand der besagten Person erkennt.

10. System (1) nach Patentanspruch 9, **wobei** die besagte tragbare Vorrichtung mit ersten drahtlosen Kommunikationsmitteln (8) versehen ist, wobei die besagte elektronische Steuereinheit (9) zu einer Vorrichtung (13) gehört, die sich von der besagten tragbaren Vorrichtung (2) unterscheidet und mit zweiten drahtlosen Kommunikationsmitteln (14) versehen ist, wobei die besagte tragbare Vorrichtung (2) dazu konfiguriert ist, die besagte mindestens eine EEG-Spur durch die besagten ersten und zweiten drahtlosen Kommunikationsmittel (8, 14) an die besagte elektronische Vorrichtung (13) zu übertragen, sobald die besagte mindestens eine EEG-Spur erfasst wurde.

## Revendications

1. Procédé mis en œuvre par ordinateur pour détecter un état d'esprit de micro-sommeil d'une personne, où le micro-sommeil désigne l'état physique d'une personne qui commence lorsque la paupière supérieure d'une personne s'abaisse jusqu'à recouvrir complètement la pupille et que ce recouvrement est maintenu pendant au moins 0,5 seconde, et pour avertir ladite personne dans le cas positif, ladite détection étant mise en œuvre en traitant au moins un tracé EEG de ladite personne au moyen d'au moins un réseau neuronal convolutif convenablement entraîné, **où**:
ledit procédé comprend une étape d'entraînement comprenant les opérations suivantes:
- recevoir un ensemble de tracés EEG d'entraînement comprenant une pluralité de tracés EEG acquis sur plus d'une personne par au moins une électrode placée en position frontopolaire FP1 ou FP2 sur le cuir chevelu de ladite plus d'une personne;
- une étape de prétraitement desdits tracés EEG d'entraînement consistant à filtrer lesdits tracés EEG d'entraînement au moyen d'un filtre passe-haut dont la fréquence de coupure est choisie entre 0,001 et 0,05 Hz et/ou d'un filtre passe-bas dont la fréquence de coupure est choisie entre 10 et 25 Hz;
- recevoir, pour chacun desdits tracés EEG d'entraînement, une annotation indiquant l'identification, pour au moins une partie dudit tracé EEG d'entraînement, dudit état d'esprit de micro-sommeil destiné à être détecté;
- fournir chacun desdits tracés EEG d'entraînement et ladite annotation relative comme données d'entrée dudit réseau neuronal convolutif, ledit réseau neuronal convolutif se terminant par une fonction d'activation sigmoïde, afin d'obtenir un signal de sortie de classification dans lequel la probabilité de la détection dudit état d'esprit de micro-sommeil dans ledit tracé EEG d'entraînement d'entrée est enregistrée;
- au moyen dudit réseau neuronal convolutif, en traitant chacun desdits tracés EEG d'entraînement et en entraînant ledit réseau neuronal convolutif, en comparant ledit signal de sortie de classification avec l'annotation relative audit tracé EEG d'entraînement;
**et où** ledit procédé comprend également une étape de classification d'au moins un tracé EEG acquis sur une personne par au moins une électrode disposée en position frontopolaire FP1 ou FP2 sur le cuir chevelu de ladite personne, de manière à détecter ou non ledit état d'esprit de micro-sommeil chez ladite personne, ladite étape de classification comprenant les opérations suivantes:
- recevoir au moins ledit tracé EEG;
- une étape de prétraitement dudit tracé EEG comprenant le filtrage dudit tracé EEG au moyen d'un filtre passe-haut dont la fréquence de coupure est choisie entre 0,001 et 0,05 Hz et/ou d'un filtre passe-bas dont la fréquence de coupure est choisie entre 10 et 25 Hz;
- fournir ledit tracé EEG comme entrée audit réseau neuronal convolutif, après que ledit réseau neuronal convolutif a été entraîné;
- traiter ledit tracé EEG au moyen dudit réseau neuronal convolutif déjà entraîné;
- recevoir ledit signal de sortie de classification pour ledit tracé EEG à partir de ladite fonction d'activation, et décider de détecter ou non ledit état d'esprit de micro-sommeil de ladite personne sur la base desdits signaux de sortie de classification.

2. Procédé mis en œuvre par ordinateur selon la revendication 1, **où** ladite étape de classification, en particulier ladite décision de détecter ou non ledit état d'esprit de micro-sommeil de ladite personne, est basée sur lesdits signaux de sortie de classification obtenus par le traitement dudit tracé EEG acquis par deux électrodes disposées en position frontopolaire FP1 et en position frontopolaire FP2 sur le cuir chevelu de ladite personne.

3. Procédé mis en œuvre par ordinateur selon l'une quelconque des revendications précédentes, **où** ledit réseau neuronal convolutif est un réseau du type "Réseau convolutif temporel".

4. Procédé mis en œuvre par ordinateur selon l'une quelconque des revendications précédentes, **où** ladite opération consistant à décider de détecter ou non l'état d'esprit de micro-sommeil d'une personne est mise en œuvre par au moins un réseau neuronal récurrent disposé en aval dudit au moins un réseau neuronal convolutif, de sorte que ledit réseau neuronal récurrent reçoit en entrée lesdits signaux de sortie de classification générés par ledit réseau neuronal convolutif.

5. Procédé mis en œuvre par ordinateur selon l'une quelconque des revendications précédentes, **où** ladite opération consistant à décider de détecter ou non ledit état d'esprit de micro-sommeil d'une personne dans ledit tracé EEG consiste à vérifier si la probabilité de détecter ledit état d'esprit de micro-sommeil indiqué dans ledit signal de sortie de classification est supérieure à un seuil prédéfini pendant une période de temps minimale prédéfinie.

6. Procédé mis en œuvre par ordinateur selon l'une quelconque des revendications précédentes, **où** chacune desdites annotations desdits tracés EEG d'entraînement comprend un premier vecteur consistant en un nombre de cellules de mémoire égal à la durée de chacun desdits tracés EEG d'entraînement divisée par une plage d'échantillonnage prédéfinie, où dans chacune desdites cellules de mémoire la présence ou l'absence dudit état d'esprit de micro-sommeil d'une personne est notée pour chacune des plages d'échantillonnage dudit tracé EEG d'entraînement.

7. Procédé mis en œuvre par ordinateur selon l'une quelconque des revendications précédentes, **où** ledit signal de sortie de classification comprend un deuxième vecteur comprenant un nombre de cellules de mémoire égal à la durée de chacun desdits tracés EEG divisée par une plage d'échantillonnage prédéfinie, où dans chacune desdites cellules de mémoire la probabilité d'identifier ledit état d'esprit de micro-sommeil d'une personne à une plage d'échantillonnage spécifique dudit tracé EEG est enregistrée.

8. Procédé mis en œuvre par ordinateur selon l'une des revendications 6 ou 7, **où** ladite plage d'échantillonnage est comprise entre 5 ms et 50 ms, de préférence ladite plage d'échantillonnage est choisie pour être de 10 ms.

9. Système (1) de détection de l'état d'esprit d'une personne (P) par le traitement d'au moins un tracé EEG de ladite personne (P) selon le procédé de l'une quelconque des revendications précédentes, **où** il comprend:
- un dispositif (2) portable par ladite personne (P) de sorte que ledit dispositif portable (2) soit placé au moins au niveau de la partie avant du cuir chevelu de ladite personne (P), ledit dispositif portable (2) étant doté d'au moins une électrode (3, 4) adaptée pour être mise en contact avec le cuir chevelu de ladite personne (P) dans la position frontopolaire FP1 ou FP2 du même cuir chevelu de ladite personne (P), ledit dispositif portable (2) étant configuré pour acquérir au moins ledit tracé EEG de ladite personne (P) au moyen de ladite électrode (3, 4);
- une unité électronique de commande (9) comprenant des moyens de stockage (10) dans lesquels un réseau neuronal convolutif est stocké selon l'une quelconque des revendications précédentes et des moyens de traitement (11) configurés pour exécuter ledit réseau neuronal convolutif, ledit réseau neuronal convolutif étant configuré pour exécuter:
- ladite étape d'entraînement, lorsque ledit réseau neuronal convolutif reçoit en entrée lesdits tracés EEG d'entraînement et leurs annotations; et/ou
- ladite étape de classification, lorsque ledit réseau neuronal convolutif reçoit en entrée au moins un tracé EEG de ladite personne (P) de manière à détecter ou non ledit état d'esprit spécifique de micro-sommeil chez ladite personne;
- des moyens d'alerte, configurés pour avertir ladite personne de la détection dudit état d'esprit de micro-sommeil lorsque ladite unité électronique de commande détecte ledit état d'esprit de micro-sommeil de ladite personne.

10. Système (1) selon la revendication 9, **où** ledit dispositif portable est doté de premiers moyens de communication sans fil (8), ladite unité électronique de commande (9) appartenant à un dispositif (13) distinct dudit dispositif portable (2) et doté de deuxièmes moyens de communication sans fil (14), ledit dispositif portable (2) étant configuré, une fois que ledit au moins un tracé EEG est acquis, pour transférer ledit au moins un tracé EEG audit dispositif électronique (13) par lesdits premiers et deuxièmes moyens de communication sans fil (8, 14).
